Europäisches Patentamt

European Patent Office

Office européen des brevets

(11) Numéro de publication: **0 419 343 A1**

## (12) DEMANDE DE BREVET EUROPEEN

(21) Numéro de dépôt: 90402571.5

(22) Date de dépôt: 18.09.90

(51) Int. Cl.5: **A61B 1/00**

(30) Priorité: 20.09.89 FR 8912359

(43) Date de publication de la demande:
27.03.91 Bulletin 91/13

(84) Etats contractants désignés:
**CH DE GB IT LI**

(71) Demandeur: **Croisy, Renaud**
**15 rue Marignac**
**CH-1206 Geneve(CH)**

Demandeur: **Croisy, Nicolas**
**15 rue Marignac**
**CH-1206 Geneve(CH)**

(72) Inventeur: **Croisy, Renaud**
**15 rue Marignac**
**CH-1206 Geneve(CH)**
Inventeur: **Croisy, Nicolas**
**15 rue Marignac**
**CH-1206 Geneve(CH)**

(74) Mandataire: **Mongrédien, André et al**
**c/o SOCIETE DE PROTECTION DES**
**INVENTIONS 25, rue de Ponthieu**
**F-75008 Paris(FR)**

(54) Catheter pour la vision dans un conduit, notemment du corps humain.

(57) Cathéter pour la vision dans un conduit, notamment du corps humain, comportant une fibre optique annulaire d'éclairage (2) et une fibre optique d'observation (8) coaxiale à la précédente et munie à son extrémité d'une lentille (10), caractérisé en ce que la fixation de la lentille (10) à l'extrémité de la fibre d'observation (8) est réalisée par sertissage à l'aide d'un ensemble de deux bagues métalliques (14, 16) concentriques dont la première ou bague centrale (14), de forme annulaire cylindrique, est sertie sur la lentille (10) et l'extrémité de la fibre d'observation (8) et dont la seconde (16), également annulaire, est sertie, au même niveau longitudinal que la première, sur la fibre optique d'éclairage annulaire (2) disposée autour de la fibre d'observation (8), cette seconde bague (16) présentant un épaulement (18) dirigé vers l'arrière du cathéter et destiné à maintenir en translation la gaine externe (12) en matière plastique du cathéter.

FIG. 1

EP 0 419 343 A1

## CATHETER POUR LA VISION DANS UN CONDUIT, NOTAMMENT DU CORPS HUMAIN

La présente invention a trait, d'une façon générale mais non exclusive, au domaine médical de l'observation du corps humain à l'aide de sondes endoscopiques que l'on introduit dans un conduit interne naturel ou artificiel de ce corps.

Elle s'applique tout particulièrement aux cathéters miniaturisés, d'emploi de plus en plus fréquent aujourd'hui, dont les dimensions diamétrales sont au plus égales au millimètre.

On connait déjà des dispositifs de ce genre qui sont constitués d'une multifibre servant à l'éclairage du site à explorer et d'une multifibre d'observation qui transmet au contraire la lumière réfléchie par le site précédent vers l'observateur.

Dans des dispositifs connus de ce genre, on a déjà réalisé les deux fibres précédentes sous forme coaxiale, la fibre optique d'éclairage étant constituée par l'agglomération d'un grand nombre de monofibres dans un espace annulaire et la fibre d'observation étant constituée d'une masse cylindrique compacte de monofibres, logée au centre et dans l'axe de cet espace annulaire.

On sait que, pour obtenir un champ de vision acceptable à l'aide d'une telle fibre d'observation, composée par exemple de 2 000 à 3 000 monofibres, il est nécessaire de prévoir une lentille optique en verre, généralement constituée par un cylindre de verre, à l'extrémité de la multifibre d'observation, faute de quoi la vision ne serait que ponctuelle sous la forme d'un nombre de points très localisés correspondant chacun à l'information transmise par l'une des monofibres.

Jusqu'à ce jour, on a tenté, de façon toute naturelle, de fixer cette lentille cylindrique par collage à l'extrémité de la fibre d'observation. Or, aucune des colles utilisées jusqu'à maintenant, telles que l'Araldite par exemple, n'est capable d'assurer un collage définitif sérieux. Au contraire, tôt ou tard, la lentille optique d'un tel système d'observation finit par se séparer de la fibre et le cathéter n'est plus utilisable.

Il existe encore un autre problème technique non résolu aujourd'hui dans le domaine de telles fibres d'observation. En effet, il est absolument impératif pour les besoins du fonctionnement d'un tel cathéter, qu'il soit revêtu extérieurement d'une gaine en plastique, le plus souvent d'ailleurs constituée de polyamide. Cette gaine a besoin d'être fixée sur le cathéter, car sinon elle glisse sur celui-ci au fur et à mesure de ses mouvements et peut, le cas échéant, venir masquer la lentille.

Pour fixer cette gaine à l'extérieur de la fibre d'éclairage, l'idée immédiate qui vient à l'esprit consiste également à la coller sur cette fibre. Or, il n'existe pas dans le commerce à l'heure actuelle de colle permettant une bonne fixation de cette gaine extérieure tout en étant bio-compatible avec le milieu vivant dans lequel le cathéter est destiné à être introduit.

Par ailleurs, on assiste depuis plusieurs années déjà dans ce domaine technique à une évolution vers une miniaturisation de plus en plus poussée des cathéters d'endoscopie, et l'on sait réaliser ces appareils aujourd'hui avec des dimensions diamétrales inférieures à 1 mm. Dans ce domaine dimensionnel, les solutions préconisées dans l'art antérieur, par exemple dans le DE-A-3 718 609 et prévues pour des cathéters de l'ordre de 3 à 5 mm de diamètre, ne sont plus applicables.

Dans ce document en effet, les figures 1 et 4 montrent une réalisation comportant deux familles de fibres optiques coaxiales, l'une au centre pour la vision et l'autre, à la périphérie, pour l'éclairage. Une lentille centrale 16 permet le fonctionnement de la fibre de vision et des lentilles 21 permettent l'éclairage correct par les fibres correspondantes. Les deux familles de fibres concentriques sont maintenues par des tubes ou bagues qui les entourent, mais les différentes fixations sont réalisées par collage, et, en ce qui concerne les fibres d'éclairage 11 par un ciment (colonne 5, lignes 33 à 38) avec un jeu diamétral par rapport au tube 15 maintenant la lentille 16. Une telle disposition est parfaitement adaptée à des cathéters de 3 à 5 mm de diamètre.

En revanche, avec des cathéters de dimensions diamétrales égales ou inférieures au millimètre, l'enseignement précédent est inapplicable, car à ce niveau de miniaturisation il est impossible d'employer la colle sans qu'elle déborde et ne vienne recouvrir en le colorant le faisceau de vision et/ou d'éclairage, causant ainsi un trouble général rédhibitoire de la qualité optique de l'appareil. Il en est de même de la colle que l'on chercherait à utiliseer pour fixer la gaine externe de téflon ou polyamide en translation sur le cathéter, laquelle, de surcroît ne tiendrait pas collée, et qui, sous l'influence de la chaleur corporelle subie lors de l'utilisation de l'appareil, se dilaterait et finirait également par venir masquer la fibre de vision.

La présente invention a pour objet un cathéter pour la vision dans un conduit qui permet, à l'aide de moyens particulièrement simples à mettre en oeuvre, à la fois de fixer sans colle la lentille optique à l'extrémité de la fibre d'observation et de fixer la gaine en plastique externe en translation par rapport au cathéter.

Ce cathéter pour la vision dans un conduit, notamment du corps humain, comportant une fibre optique annulaire d'éclairage et une fibre optique d'observation coaxiale à la précédente et munie à son extrémité d'une lentille, se caractérise en ce que la fixation de la lentille à l'extrémité de la fibre d'observation est réalisée par sertissage à l'aide d'un ensemble de deux bagues métalliques concentriques, par exemple en acier chrome nickel, dont la première ou bague centrale, de forme annulaire cylindrique, est sertie sur la lentille et l'extrémité de la fibre d'observation et dont la seconde, également annulaire, est sertie, au même niveau longitudinal que la première, sur la fibre optique d'éclairage annulaire disposée autour de la fibre d'observation, cette seconde bague présentant un épaulement dirigé vers l'arrière du cathéter et destiné à fixer en translation la gaine externe en matière plastique du cathéter.

Le sertissage sans colle de la lentille d'observation et la fixation en translation de la gaine externe en plastique du cathéter sont ainsi réalisés simultanément grâce à l'utilisation de deux bagues annulaires concentriques serties l'une sur l'autre.

De toute façon, l'invention sera mieux comprise à l'aide de la lecture qui suit d'un exemple de mise en oeuvre, lequel sera décrit en se référant aux figures schématiques 1 et 2 sur lesquelles :

- la figure 1 est une vue en coupe axiale d'un cathéter selon l'invention ;

- la figure 2 est une coupe selon AB du dispositif de la figure 1.

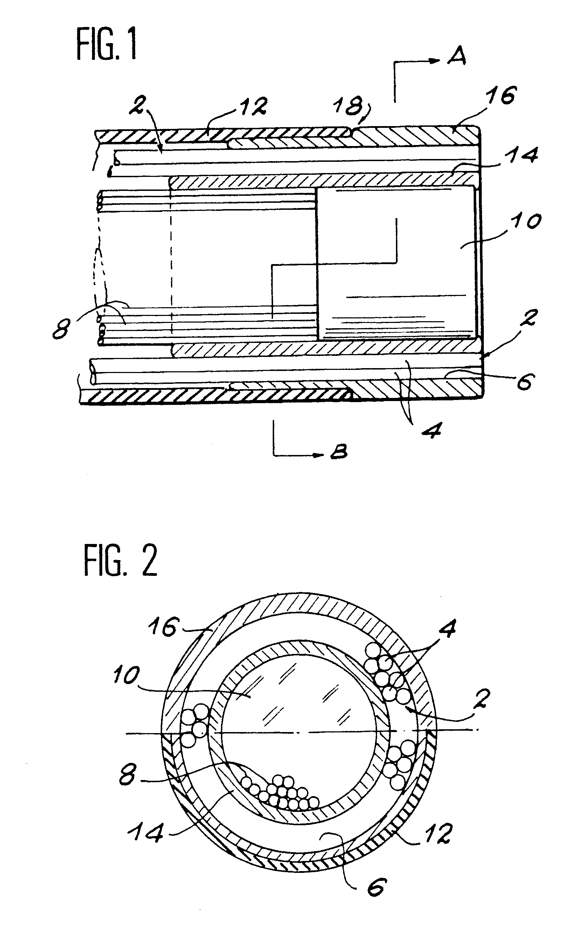

Le cathéter représenté à titre non limitatif sur les figures 1 et 2 comporte d'abord une fibre d'éclairage 2 constituée par la juxtaposition d'un grand nombre de monofibres telles que 4 sur la figure 2 dans un espace annulaire 6. Cette multifibre d'éclairage est réalisée par recouvrement de la fibre de vision 8 que l'on va décrire maintenant, à l'aide d'un véritable tapissage de monofibres entassées les unes contre les autres sans collage ni liant.

La fibre d'observation 8, d'un diamètre de l'ordre de 0,35 mm dans l'exemple décrit, est également une multifibre comportant un nombre de brins de l'ordre de 2 500. Comme expliqué précédemment, elle est complétée, à son extrémité, par la lentille optique 10 qui est un simple cylindre de verre de même rayon que la multifibre 8. C'est grâce, rappelons-le, à cette lentille 10 que l'observateur, à l'autre extrémité non représentée du cathéter, peut avoir une vision claire et complète dans un champ optique relativement important.

Le cathéter précédent est enfin complété par une gaine externe 12 en polyamide destinée à assurer un contact physiquement et physiologiquement satisfaisant avec le conduit interne du corps vivant dans lequel il est introduit.

Conformément à l'invention, deux bagues métalliques concentriques, à savoir la bague centrale 14 et la bague extérieure 16 assurent, comme on va l'expliquer maintenant, par simple sertissage, la fixation d'une part de la lentille 10 sur la multifibre d'observation 8 et d'autre part de l'enveloppe en polyamide 12 sur le cathéter.

En effet, la bague interne ou centrale 14, de forme annulaire cylindrique, est sertie en enserrant à la fois la lentille 10 et l'extrémité de la fibre d'observation 8 qui sont ainsi fixées solidement l'une à l'autre. Par ailleurs, la seconde bague 16, également annulaire et située par rapport à l'axe du cathéter au même niveau longitudinal que la première bague centrale 14; elle possède un épaulement 18, dirigé vers la face arrière du cathéter, et elle est sertie à son tour sur la périphérie externe de la fibre d'éclairage 2, assurant ainsi la fixation de cette fibre d'éclairage 2 et complétant par là-même en même temps le sertissage de la première bague centrale 14. Quant à l'épaulement 18, il sert de butée en translation à l'enveloppe de plastique 12 qui est ensuite enfilée à l'extérieur du système et dont l'extrémité avant vient ainsi en butée contre cet épaulement 18 de la deuxième bague externe 16.

On voit ainsi qu'à l'aide des moyens très simples de l'invention, on résout à la fois les deux problèmes non résolus jusqu'à ce jour qu'étaient la fixation sans colle de la lentille 10 sur la fibre d'observation 8 et la fixation en translation de l'enveloppe externe 12. Celle-ci occupe ainsi, par rapport au reste du cathéter, une place fixe indépendante des mouvements d'introduction ou de retrait de ce même cathéter dans le conduit à observer.

## Revendications

1. Cathéter pour la vision dans un conduit, notamment du corps humain, comportant une fibre optique annulaire d'éclairage (2) et une fibre optique d'observation (8) coaxiale à la précédente et munie à son extrémité d'une lentille (10), caractérisé en ce que la fixation de la lentille (10) à l'extrémité de la fibre d'observation (8) est réalisée par sertissage à l'aide d'un ensemble de deux bagues métalliques (14, 16) concentriques dont la première ou bague centrale (14), de forme annulaire cylindrique, est sertie sur la lentille (10) et l'extrémité de la fibre d'observation (8) et dont la seconde (16), également annulaire, est sertie, au même niveau longitudinal que la première, sur la fibre optique d'éclairage annulaire (2) disposée autour de la fibre d'observation (8), cette seconde bague (16) présentant un épaulement (18) dirigé vers l'arrière du cathéter et destiné à maintenir en translation la gaine externe (12) en matière plastique du cathéter.

# FIG. 1

# FIG. 2

Office européen
des brevets

RAPPORT DE RECHERCHE
EUROPEENNE

Numéro de la demande

EP 90 40 2571

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-4 790 295  (Y. TASHIRO)<br>* abrégé * * colonne 3, ligne 60 - colonne 4, ligne 41; figures * | 1 | A 61 B 1 00 |
| Y | DE-A-3 637 789  (OLYMPUS OPTICAL CO.)<br>* le document en entier * | 1 | |
| D,A | DE-A-3 718 609  (OLYMPUS OPTICAL CO.)<br>* colonne 5, lignes 17 - 42; figures * | 1 | |
| A | EP-A-0 100 517  (SUMIMOTO ELECTRIC INDUSTRIES LIM.)<br>* page 18, ligne 1 - page 21, ligne 14: figures 13, 14 * | 1 | |

|  | DOMAINES TECHNIQUES RECHERCHES (Int. Cl.5) |
|---|---|
|  | A 61 B |

Le présent rapport de recherche a été établi pour toutes les revendications

| Lieu de la recherche | Date d'achèvement de la recherche | Examinateur |
|---|---|---|
| La Haye | 28 novembre 90 | FERRIGNO, A. |